# EUROPEAN PATENT SPECIFICATION

(11) **EP 3 079 743 B1**
(45) Date of publication and mention of the grant of the patent: **03.08.2022**
(21) Application number: 14868882.3
(22) Date of filing: 10.12.2014
(51) Int. Cl.: A61M 11/02, A61M 11/06, A61M 15/00, A61M 16/00, A61M 16/06, A61M 16/14, A61M 16/20, A61K 9/00, A61K 31/137, A61K 31/4166, A61K 31/485, A61K 31/5517, A61K 31/7004, A61P 9/04, A61P 3/00, A61P 37/00, A61M 16/04

(54) **APPARATUS**
VORRICHTUNG
APPAREIL

(30) Priority: 11.12.2013 AU 2013904823
(43) Date of publication of application: 19.10.2016
(73) Proprietor: De Motu Cordis Pty. Ltd., Wilston, Queensland 4051 (AU)
(72) Inventor: FRASER, John Francis, Wilston, Queensland 4051 (AU); GREGORY, Shaun David, Murrumba Downs 4503 (AU)
(74) Representative: Onsagers AS
(86) International application number: PCT/AU2014/050406
(87) International publication number: WO 2015/085362

(56) References cited:
- EP-A2- 0 826 386
- EP-A2- 1 655 050
- WO-A1-97/37708
- WO-A1-2006/075184
- WO-A2-2005/065756
- WO-A2-2007/093310
- US-A- 6 003 512
- US-A1- 2002 144 680
- US-A1- 2006 292 082
- US-A1- 2008 135 047
- US-A1- 2011 126 830
- US-A1- 2011 308 516
- US-A1- 2012 111 324
- US-A1- 2012 138 049
- US-A1- 2013 213 397
- US-A1- 2013 253 065
- ASTHMA SOCIETY OF CANADA: 'How to use a Diskhaler' 28 January 2015, XP055348835 Retrieved from the Internet: <URL:http://www.asthma.ca/adults/treatment/ diskhaler.php>

## Description

### Background of the Invention

The present invention relates to an apparatus configured to administer microparticles of adrenalin to the lungs of a biological subject to treat anaphylaxis or cardiac arrest.

### Description of the Prior Art

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

Cardiac arrest is a significant cause of mortality, with as many as 500,000 deaths reported in the USA each year and almost 90% of cases occurring at home or in the community. Survivors frequently have devastating brain injury due to the variable period of brain hypoxia suffered. Current data shows survival post cardiac arrest is 24-40% in hospital vs. 9% survival in community. "Survival" is not an acceptable outcome - it must be useful survival. The difference is largely attributable to delays in return of cerebral perfusion. Unlike most other organs, the brain has minimal metabolic reserve or ability to generate energy anaerobically, and thus irreversible damage commences within seconds of cardiac arrest. It is estimated that if new strategies could reduce cardiac arrest mortality by 10%, this would reduce the cost incurred through cardiac arrests by $75 billion.

It is known to administer drugs such as adrenalin and atropine to subjects in cardiac arrest, and in particular during cardiopulmonary resuscitation (CPR).

Conventional CPR is a known technique which involves performing chest compressions on, and supplying artificial breaths (also known as rescue breaths) to, the subject. In this regard, the Australian Resuscitation Council recommends that CPR be performed using a 30:2 compression to ventilation ratio with minimal interruptions. The Council further recommends that 1 mg of adrenalin be administered to subjects with a shockable heart rhythm after every second shock and every two CPR loops thereafter. For subjects with non-shockable heart rhythms, it is recommended that 1 mg of adrenalin be administered immediately and every two CPR loops thereafter. However, these doses could be subject to variation.

In this regard, CPR temporarily maintains blood flow to the brain thus preserving brain function while medical treatment is sought. The additional administration of adrenalin is known to strengthen cardiac contractions, increase macrocirculatory pressure, and thus increase survival rates, and in particular increase post-resuscitation quality of life of subjects, for example, by decreasing the risk of severe permanent disability post-resuscitation.

However delivery of adrenalin typically involves either intravenous injection, or administering a dose of liquid adrenalin into the subject's trachea. In this regard, if a single operator is performing CPR on a subject, it will be necessary to interrupt the compressions and ventilation in order to administer the adrenalin which is contrary to the best practices outlined above, and can adversely affect the subject. In addition, the administration of adrenalin using any of the abovementioned methods requires considerable medical training, and therefore typically cannot be performed in circumstances where trained medical personnel are not present, severely impacting the subject's chances for recovery and a higher quality of life post-resuscitation. Both methods also have a number of further disadvantages.

The former method requires access to a vein in order to inject the adrenalin, which can be particularly problematic for subjects with heart dysfunction, where veins are difficult to access due to little or no blood circulation. In the event it is possible to access a vein, the lack of circulation also prevents any injected adrenalin from travelling to the cardiac tissue, and as a result it is typically necessary to further administer intravenous saline in order to encourage circulation of the drug. Furthermore, it can be particularly difficult to gain access to a vein if the subject is a child or infant.

The latter technique typically involves pouring a dose of liquid adrenalin into the subject's trachea. However, this method usually results in less active ingredient being ultimately delivered to the subject, as only a small proportion of the dose flows to the alveoli where it can be subsequently absorbed.

In addition, it is recommended that adrenalin be administered immediately in the event of cardiac arrest, and furthermore that adrenalin be administered until a normal rhythm is restored in accordance with the abovementioned practice guidelines. In this regard, any time spent finding an appropriate vein and waiting for the blood to circulate to the heart under the influence of saline, or waiting for a liquid dose to flow into the alveoli, perfuse into the blood stream and travel to the heart, can adversely impact a subject's expected outcome.

Other methods of adrenalin administration include intraosseous infusion, administration via a central line, and the like. However, such methods typically require advanced medical training and specialist apparatus.

Delivery of adrenalin in dry-powder form to the lungs of a subject by using a positive pressure gas flow is known from EP 0826386 A2, WO 97/37708 A1, and from US 2012/0111324 A1 for treatment of cardiac arrest. Delivery of adrenalin in dry-powder form to the lungs of a subject for the treatment of anaphylaxis is known from US 2013/0253065 A1. Another device for delivery of adrenalin in dry-powder form to the lungs of a subject is known from WO 2007/093310 A2.

### Summary of the Present Invention

The present invention seeks to ameliorate one or more of the problems associated with the prior art.

The invention is defined by the subject-matter of claim 1.

### Brief Description of the Drawings

An example of the present invention will now be described with reference to the accompanying drawings, in which: -
Figure 1A is a schematic diagram of a front view of a first example of an apparatus for use in administering a particulate medicament to subject's airway;
Figure 1B is a schematic diagram of a back view of the apparatus of Figure 1A;
Figure 1C is a schematic diagram of a side view of the apparatus of Figure 1A;
Figure 1D is a schematic diagram of a perspective view of the apparatus of Figure 1A;
Figure 2A is a schematic diagram of a perspective view of a second example of an apparatus for use in administering a particulate medicament to a subject's airway;
Figure 2B is a schematic diagram of a front view of the apparatus of Figure 2A;
Figure 2C is a schematic diagram of a side view of the apparatus of Figure 2A;
Figure 2D is a schematic diagram of a plan view of the apparatus of Figure 2A;
Figure 2E is a schematic diagram of an underside view of the apparatus of Figure 2A;
Figure 3A is a schematic diagram of a side view of the apparatus of Figure 2A, in use;
Figure 3B is a schematic diagram of a side view of the apparatus of Figure 2A, in use;
Figure 4A is a schematic diagram of a perspective view of a further example of an apparatus for use in administering a particulate medicament to a subject's airway;
Figure 4B is a schematic diagram of a plan view of the apparatus of Figure 4A;
Figure 4C is a schematic diagram of a side view of the apparatus of Figure 4A;
Figure 4D is a schematic diagram of a front view of the apparatus of Figure 4A;
Figure 5A is a schematic diagram of a plan view of a further example of an apparatus for use in administering a particulate medicament to a subject's airway;
Figure 5B is a schematic diagram of a side view of the apparatus of Figure 5A;
Figure 5C is a schematic diagram of a perspective view of the apparatus of Figure 5A;
Figure 6A is a schematic diagram of a plan view of a further example of an apparatus for use in administering a particulate medicament to a subject's airway;
Figure 6B is a schematic diagram of a side view of the apparatus of Figure 6A;
Figure 6C is a schematic diagram of a perspective view of the apparatus of Figure 6A
Figure 7A is a schematic diagram of a plan view of a further example of an apparatus for use in administering a particulate medicament to a subject's airway;
Figure 7B is a schematic diagram of a side view of the apparatus of Figure 7A;
Figure 7C is a schematic diagram of a perspective view of the apparatus of Figure 7A;
Figure 7D is a schematic diagram of an end view of the apparatus of Figure 7A;
Figure 7E is a schematic diagram of an exploded view of the apparatus of Figure 7A;
Figure 7F is a schematic diagram of a cut-away view of the apparatus of Figure 7A along the line A-A';
Figure 7G is a schematic diagram of a cut-away view of the apparatus of Figure 7A along the line B-B';
Figure 7H is a schematic diagram of a cut-away view of the apparatus of Figure 7A in the loading position;
Figure 7I is a schematic diagram of a cut-away view of the apparatus of Figure 7A in the dispensing position;
Figure 8A is a schematic diagram of an end view of a further example of an apparatus for use in administering a particulate medicament to a subject's airway;
Figure 8B is a schematic diagram of a side view of the apparatus of Figure 8A;
Figure 8C is a schematic diagram of a plan view of the apparatus of Figure 8A;
Figure 8D is a schematic diagram of a perspective view of the apparatus of Figure 8A;
Figure 8E is a schematic diagram of a cut-away view of the apparatus of Figure 8A along the line C-C'; and,
Figure 8F is a schematic diagram of a cut-away view of the apparatus of Figure 8A along the line D-D'; and,
Figures 9A to 9C are graphs showing the effect of delivery of particulate adrenalin to sheep on Ambulatory Blood Pressure (ABP), Heart Rate (HR) and Pulmonary Arterial Pressure (PAP), respectively.

### Detailed Description of the Preferred Embodiments

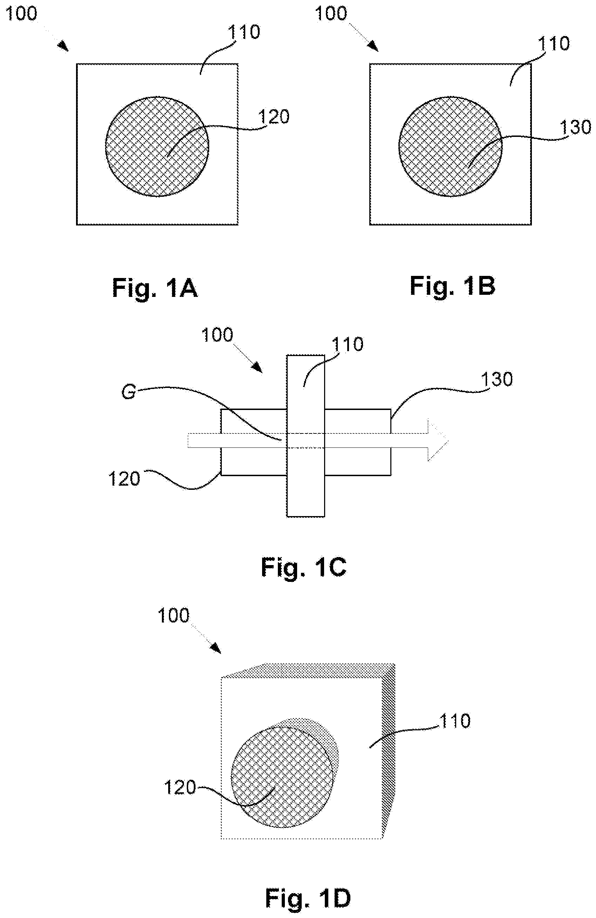

An example of an apparatus (that may be according to the invention if it has all the features of claim 1) for use in administering a particulate medicament to subject's airway will now be described with reference to Figure 1.

In this example, the apparatus 100 includes an inlet 120 that in use is in fluid communication with a supply of positive pressure gas G, and an outlet 130 that in use is in fluid communication with the subject's airway to deliver the positive pressure gas thereto. In this regard, the supply of positive pressure gas *G* may be provided by any suitable mechanism, for example, a bag-valve-mask (BVM), an Ambu bag, a manual resuscitator, an operator exhaling in fluid communication with the inlet and/or a mouthpiece or mask in communication with the inlet, a ventilator, an oxygen pump, a compressed gas supply, or the like,

The apparatus 100 further includes a medicament supply that provides a dose of particulate medicament so that the particulate medicament is entrained in a gas flow from the inlet to the outlet, thereby delivering the particulate medicament to the subject's airway.

In the current example, this includes one or more medicament containers 110 containing one or more doses of particulate medicament, the medicament container 110 being in fluid communication with the inlet and/or the outlet so that as gas *G* flows from the inlet to the outlet, the particulate medicament is entrained in the gas flow and is delivered to the subject's airway. In this regard, the medicament container 110 may include any suitable arrangement for containing the particulate medicament, such as, a compartment, a channel, a surface, or the like, and this will be discussed further below.

The particulate medicament may include any suitable medicament for administering to the subject's airways, depending upon the subject's condition and medical requirements. For example, the particulate medicament may include any one or more of adrenalin (according to the invention), glucose, or the like. In one example the particulate medicament includes microparticles (according to the invention) nanoparticles, microcapsules, nanocapsules, microspheres, and/or nanospheres of adrenalin and/or atropine for the treatment of cardiac failure, cardiac dysfunction, cardiac arrest anaphylaxis, or the like, and in another example the particulate medicament includes particulate glucose for the treatment of hypoglycaemia, diabetes induced coma or the like. It will also be appreciated that the technique can be used for a wide range of other medication such as drugs for benzodiazepine or opiate overdose such as naloxone or flumazenil, anti-epileptic drugs such as phenytoin, or the like.

Thus, this can be used to deliver particulate medicament to a biological subject by generating a gas flow using positive pressure gas, entraining a dose of particulate medicament in the gas flow and delivering the gas flow including the entrained particulate medicament to the subject's airway. It will be appreciated that, according to the invention, the gas flow delivers particulate medicament into the subject's lungs without requiring the subject to inhale.

This arrangement offers a number of significant advantages.

In particular, the apparatus 100 provides a very fast delivery mechanism for administering a medicament to a subject, which can significantly improve subject outcomes. For example, for subjects with cardiac dysfunction, the ability to more rapidly administer adrenalin, particularly when compared with traditional methods such as intravenous administration, significantly increases survival rates and decreases the risk of severe post-resuscitation disability.

Similarly in the case of hypoglycaemia and diabetes induced comas, it is important to deliver glucose as quickly as possible, whilst this often is not possible orally due to the inability of the subject to swallow, as well as the presence of vomit or the like, whilst the subject is often unable to inhale, meaning positive delivery of the medicament is required. In the case of drug overdoses, such as opiates, benzodiazepines or heroin, rapid delivery of appropriate drugs reduces risk of patients suffering hypoxic brain injury, whilst aspiration with anti-epileptic drugs can be used to stop seizures and reduces the risk of brain injury, whilst reducing the difficulty of delivery drugs to a fitting patient.

The administration of a particulate medicament to the subject's airway also eliminates the need to use large, complex, and expensive nebulising equipment for aerosolising any liquid medicaments. Such equipment is typically only available in hospitals and specialised medical premises and requires trained personnel to setup and operate the system. In contrast, the apparatus 100 is sufficiently light-weight, portable, and inexpensive to be made available for use in many environments, such as homes, shopping centres, offices, and the like. Furthermore, the apparatus 100 may be used with any supply of positive pressure gas *G* as discussed above, such as from a bag-valve-mask (BVM), Ambu bag, mouthpiece, or simply an operator exhaling, further increasing its portability and usability.

Additionally, in this arrangement an operator may use the apparatus 100 with little or no specialised training. In this regard, the apparatus 100 could be made widely available and be readily used.

As a consequence, this allows the adrenalin to be delivered for example as part of a CPR procedure, resulting in significantly improved subject outcomes. For example, for subjects in cardiac arrest, heart failure or anaphylaxis, where the subject is not located in a hospital or specialised medical centre, the ability to have particulate adrenalin available and readily administered using the apparatus 100 during the initial stages of the condition would have a significant impact on their post-resuscitation outcomes and quality of life, as discussed above. In this regard, as it is crucial to administer CPR and adrenalin immediately in the event of a cardiac arrest, the immediate availability, simplicity and portability of the apparatus 100 increases probability of survival and decreases the risk of severe disability post-resuscitation.

Further, the use of positive pressure gas G is particularly beneficial where a subject is unresponsive, unconscious, or the like, which is typically the case for subjects with hypoglycaemia, heart failure or in cardiac arrest. In this regard, the apparatus 100 does not rely on the subject's ability to inhale, or otherwise respond, in order to administer the medicament to the subject's airways and alveoli. Rather, the positive pressure gas G entrains and pushes the particulate medicament into the airways where it is absorbed into the blood stream and rapidly circulated to the cardiac tissue. Thus, the apparatus 100 is suitable for the treatment of cardiac failure, and in one example, the apparatus 100 may be used during administration of CPR.

A method (not according to the invention) of administering CPR to a biological subject will now be described. In particular, the method includes performing at least one chest compression on the subject, and supplying at least one medicated breath. In this regard, in apparatus according to the invention, the medicated breath includes a positive pressure gas in an amount corresponding to an artificial breath, and an entrained dose of particulate medicament. The particulate medicament may include any suitable medicament depending upon the subject and their condition, such as any of the medicaments discussed above, and in one example (according to the invention) includes microparticles of adrenalin,

Further, it will be appreciated that the amount of gas corresponding to an artificial breath is an amount which is known in the art, and typically is dependent upon the size and nature of the subject. For example, the amount corresponding to an artificial breath for an infant is smaller in volume than for an adult biological subject. In addition, the amount typically also corresponds to an amount of gas supplied during a ventilation breath, also referred to as rescue breath, provided during conventional CPR and hence would be well known to the skilled person.

In particular, this method of administering CPR offers a number of significant advantages.

For example, the medicated breaths enable the subject to be simultaneously ventilated and medicated, thus an operator need not cease CPR in order to administer the medicament, and this would significantly improve the subject's prognosis. Furthermore, as the particulate medicament is delivered while entrained in the positive pressure gas flow, it will rapidly flow through the subject's airways into the alveoli where it is rapidly absorbed into the blood stream, and circulate to the target organ(s).

In instances where the subject has undergone cardiac arrest, performing CPR including supplying medicated breaths provides a mechanism to rapidly deliver adrenalin to the subject's blood stream, via the lungs, and from the blood stream to cardiac tissue. Furthermore, the subject may be simultaneously ventilated and medicated with adrenalin, thus enabling a single operator to perform both functions without having to interrupt the CPR, which in turn increases the subject's chances of survival as well as decreasing the risk of severe disability postresuscitation.

In addition, a plurality of doses of particulate adrenalin may be administered to the subject via multiple medicated breaths during CPR. In this regard, as one medicated breath typically corresponds to one dose of particulate adrenalin, as more medicated breaths are supplied to the subject's airways, more doses of adrenalin are delivered to the cardiac tissue and thus the subject's prognosis is significantly improved. Hence, the method may include performing the chest compressions and supplying the medicated breaths in any suitable ratio, for example a ratio of any one of 30:2, 30:1, and 60:1, or any other suitable amount as current best practice dictates.

However, it will be appreciated that the method may also include one or more non-medicated breaths, for example, supplying positive pressure gas in an amount corresponding to an artificial breath without an entrained dose of particulate medicament, similar to known ventilation breaths provided during conventional CPR. In this regard, the method of performing CPR may include performing 30 chest compressions, supplying two non-medicated breaths, performing 30 chest compressions, supplying one medicated breath and one non-medicated breath, and repeating the above steps. However, this is not essential and any combination of performing chest compressions, and supplying medicated and non-medicated breaths may be performed.

Furthermore, the method may be performed using the apparatus 100, 200 of any of the examples provided herein. In this regard, an operator does not require specialist training, and in addition the apparatus is sufficiently simple and relatively small to be made available in any environment outside of specialised medical centres, such as in the home, ambulance, shopping centres, public buildings, and the like. Thus, by performing this method any subject requiring CPR outside of a hospital or medical centre will have a greatly improved prognosis in terms of both survival and post-resuscitation quality of life.

A number of further features will now be described.

In one example, the apparatus 100 may administer different doses of particulate medicament, depending upon one or more characteristics of the subject, for example whether the subject is an adult, a child, an infant, a male, a female, the subject's body weight and the like. This may be achieved in any suitable manner, and in one example the apparatus 100 may include a number of medicament containers 110, each including a different dose, such that the operator manually selects a medicament container 110 including the appropriate dose to be administered based upon one or more of the subject's characteristics. In a further example, the apparatus 100 may selectively deliver a dose of particulate medicament according to the subject's characteristics, for example, using an electronic processing device, and this will be discussed further below. In this regard, the electronic processing device may automatically ascertain the subject's characteristics or alternatively an operator may select the characteristics using an input device.

Alternatively, a number of different apparatus 100 may be provided, each including medicament containers 110 that include a dose of particulate medicament corresponding to predetermined Subject characteristics. For example, an adult, a child and an infant model of the apparatus 100 may be provided where each model includes one or more doses appropriate to an adult, a child and an infant, respectively. In a further example, the different models of apparatus 100 may be provided based upon ranges of body weight.

In one example, the medicament container 110 of the apparatus 100 may include at least one rupturable membrane that, when ruptured, allows the gas to flow through the medicament container 110 to thereby entrain the particulate medicament In this respect, the rupturable membrane may form at least a portion of one or more walls of the medicament container 110. Furthermore, the rupturable membrane may be ruptured, in use, in any suitable manner, such as under the influence of the positive pressure gas *G*, actuation of a piercing member or peeling member prior to or during supply of the gas, or the like, and this will be discussed further below. Alternatively, the medicament container 110 could be sealed using any releasable sealing means that can be pressure actuated, such as pressure operated valves or flaps, or the like.

In particular, the rupturable membrane offers a number of advantages, including sealing the particulate medicament from the external environment until the medicament is to be administered to the subject. This decreases the risk of contamination or degradation from the external environment and ensures the medicament can be safely transported and stored for a predetermined amount of time. In addition, the rupturable membrane provides the convenience and usability of allowing the medicament to be dispensed from the medicament container following rupture of the membrane, rather than requiring an operator locate and measure the medicament separately and then provide the correct dose into the apparatus 100. Furthermore, the rupturable membrane saves time, thus allowing a dose of medicament to be rapidly delivered to a subject, which in turn can significantly improve the subject's outcomes.

However, the rupturable membrane is not essential and in another example the medicament container 110 may include one or more channels where the particulate medicament is provided on, or bound to, an inner surface of the channel. Thus, as positive pressure gas flows through the channel, the medicament on the inner surface is entrained in the gas flow and administered to the subject's airways, and this is discussed further below. Alternatively, the medicament container 110 may include a surface including medicament where gas flows adjacent the surface to thereby entrain medicament, or any other suitable arrangement.

In a further example, the medicament container 110 includes two rupturable membranes interleaved between the inlet 120, the medicament container 110, and the outlet 130. In this regard, the rupturable membranes form a portion of the walls of the medicament container 110 and are in fluid communication with the inlet 120 and/or outlet 130.

Furthermore, the apparatus 100 may include one or more adapters. In this regard, an inlet adaptor may be coupled to the apparatus 100 and in fluid communication with the inlet 120 and/or an outlet adaptor may be coupled to the apparatus 100 and in fluid communication with the outlet 130. In this respect, the inlet adaptor may be for coupling the apparatus 100 to one or more of a bag-valve-mask (BVM), an Ambu bag, a ventilator, an oxygen pump, a compressed gas supply, a manual resuscitator, a mouthpiece, or the like. Additionally or alternatively, the outlet adaptor may be for coupling the apparatus 100 to a mask, or an endotracheal tube, or the like. However, this is not essential and in other examples the apparatus 100 may be directly coupled and/or permanently attached to any of the above components without the use of an inlet and/or outlet adapter. Alternatively, the outlet can incorporate a mouthpiece allowing it to be placed directly into the subject's mouth.

In particular, the inlet and/or outlet adapter may include a universal adapter or universal connector. This is particularly beneficial as it allows the apparatus 100 to be provided with one or more universal adapters already attached. In this regard, in use the operator may quickly and easily select the most appropriate positive pressure gas *G* supply to be coupled to the apparatus 100 via the universal inlet adapter. Additionally or alternatively, the operator may select the most appropriate device to be coupled to the apparatus via the universal outlet adapter. In particular, the operator's selection will depend upon the circumstances, surroundings, subject's condition, and the like. For example, in a home or non-hospital setting, the positive pressure gas G may simply be supplied via the operator's exhalation, or a BVM, whereas in a hospital setting the operator may have access to a ventilator, or more complex/expensive positive pressure gas supply.

The apparatus 100 may include any number of medicament containers 110, and in one example the apparatus 100 includes a plurality of medicament containers 110, each medicament container 110 containing a dose of particulate medicament, and each container 110 being selectively connectable to at least one of the inlet 120 and outlet 130. In this regard, the apparatus 100 may allow the operator to administer a plurality of doses to the subject. For example, subjects with heart failure or in cardiac arrest may require multiple doses of medicament over a period of time in order to increase efficacy. Therefore, the apparatus 100 may be used to deliver multiple doses, thus increasing convenience, usability and subject outcomes, and this will be discussed further below.

The apparatus 100 may be provided in a number of different models for different applications. For example, an apparatus 100 including a plurality of medicament containers 110 may be for use in hospitals, medical centres, and the like, and an apparatus 100 including a single medicament container 110 may be provided in a kit, such as a first aid kit, for use in homes, offices, shopping centres, and the like. However, this feature is not essential.

In one example, the inlet 120 and the outlet 130 are mounted to a housing, and the apparatus 100 includes a rotary cartridge rotatably mounted to the housing wherein the rotary cartridge includes the plurality of medicament containers 110, the medicament containers 110 being selectively positionable in a delivery position in which the medicament container 110 is in fluid communication with at least one of the inlet 120 and outlet 130. In one example, the inlet 120 and the outlet 130 are spaced apart with the medicament container 110 provided therebetween, and thus in fluid communication with both the inlet 120 and the outlet 130. However, this is not essential and in another example, the apparatus 100 may include a Venturi constriction, where particulate medicament is entrained into the positive pressure gas flow at or near the constriction, and thus the medicament container is in fluid communication with only one of the inlet 120 or outlet 130, and this is discussed further below.

In a further example, in use, gas under negative pressure rotates the rotary cartridge to provide a next medicament container 110 in the delivery position. For example, gas under negative pressure may be supplied when a BVM re-inflates, or the like. This arrangement is particularly beneficial as the advancement of the medicament containers 110 is automatic and thus a dose of particulate medicament can be provided with each supply of an amount of positive pressure gas, for example, as medicated breaths. Thus, a plurality of doses of medicament may be administered to the subject by the operator simply, conveniently and easily, whilst ensuring the subject is administered a sufficient number of doses within the requisite timeframe in order to improve their post-resuscitation outcome.

However, this is not essential and rotation of the rotary cartridge may be achieved in any suitable manner, such as manual rotation by the operator, or rotation caused by the operator actuating an actuator, for example a button which actuates rotation. For example, these arrangements may be more preferable in situations where it is not possible, practical and/or safe to provide negative pressure gas, such as where the positive pressure gas is supplied via an operators exhalation. Thus, the apparatus 100 may include multiple mechanisms for rotating the rotary cartridge, for example, such that the cartridge rotates under the influence of negative pressure when the apparatus 100 is used with a BVM and in addition the cartridge rotates upon actuation of a button or other actuator when the apparatus 100 is supplied with an operator's exhalation.

Furthermore, actuation of the rotation of the rotary cartridge may be achieved in any suitable manner, such as using any one or more of gears, cables, vanes, and the like. In one example, the apparatus 100 includes a ratchet and pawl. In this regard, the rotary cartridge rotates in accordance with rotation of the ratchet, which in turn is controlled by the biased pawl. Thus, the negative pressure gas incident in a vane in fluid communication with the inlet 120 and/or outlet 130 may be used to release the biased pawl, thereby allowing rotation of the ratchet and hence rotary cartridge. Additionally or alternatively actuation of a button by the operator may release the biased pawl. However, this is not essential and any suitable rotation mechanism may be used.

In other examples the medicament containers 110 may be selectively positionable in a delivery position using other suitable arrangements, for example, by manually or automatically translating a medicament container 110 to the delivery position. For example, the medicament containers 110 may be linearly translated into a delivery position, for example by moving an elongate cartridge containing the medicament containers 110 linearly relative to the input 120 and/or output 130, and this will be discussed further below.

The medicament containers 110 may be positioned in any suitable arrangement with respect to each other. For example, the medicament containers 110 may be positioned at a sufficient distance from each other such that at least one medicament container 110 is at least partially in fluid communication with inlet and/or outlet during translation. It will be appreciated that this will allow gas to flow to the outlet of the apparatus 100 even in the event the apparatus 100 becomes jammed or locked during translation, and thus an operator may continue to ventilate the subject. However, this feature is not essential.

In another example it may be desirable to provide one or more non-medicated breaths interleaved with one or more medicated breaths, such as described above. In this regard, the medicated and non-medicated breaths may be provided while performing CPR. Thus, in one example the apparatus 100 may supply one or more non-medicated breaths following the supply of a medicated breath. This may be achieved in any suitable manner, and in one example the rotation of the rotary cartridge is not actuated after each medicated breath, such that a spent medicament container remains in the delivery position for one or more non-medicated breaths. In this regard, as positive pressure gas is supplied to the apparatus 100 with a spent medicament container in the delivery position, particulate medicament is not entrained in the gas flow, and hence the gas is delivered to the subject's airway as a non-medicated breath.

As mentioned above, the apparatus includes (according to the invention) include a channel extending at least part way between the inlet and the outlet to allow gas flow therethrough. This can form part of the medicament container as described above and/or can be defined by a housing of the apparatus.

The channel can include a medicament receiving surface that receives the particulate medicament prior to the particulate medicament being entrained in the gas flow. This can be performed during manufacture, or through delivery of the medicament from a supply in use. The medicament receiving surface is typically defined by a shape of the channel, such as a curvature of the channel, recess within the channel, or the like. Additionally and/or alternatively this could be defined by surface properties of at least part of a channel surface. For example, the surface could include microstructures and/or coatings for retaining the particulate medicament in place until a gas flow passes through the channel. According to the invention, the channel includes at least one baffle for generating turbulent gas flow within the channel.

According to the invention, the apparatus includes an actuator for selectively supplying a dose of particulate medicament into the gas flow, for example by delivering the dose of particulate medicament into a channel extending at least part way between the inlet and the outlet. The actuator could be of any suitable form and could include a button for actuation by an operator. This allows an operator to control when medicament, is provided to the subject, which can be used in dosage control, although this is not essential and other arrangements could be used.

The actuator includes (according to the invention) a piston for urging the particulate medicament into the channel. In one example, this can be used to deliver a single dose, or alternatively could be used to selectively delivers particulate medicament from a medicament container. In this case, the actuator can include a dosing system that receives a dose of particulate medicament from the medicament container and transfers the dose of particulate medicament to the channel, thereby controlling the amount of particular medicament delivered.

In a further example, the apparatus 100 includes a processing system. For example, a suitable processing system includes an electronic processing device, such as at least one microprocessor, a memory, an input/output device, such as one or more input keys/buttons and/or a keyboard and/or display, and an external interface interconnected via a bus. In this example the external interface can be utilised for connecting the processing system to one or more sensors and/or the rotary cartridge or other multiple dose cartridge, or one or more peripheral devices, such as an automated external defibrillator (AED), communications networks, or the like.

In use, the microprocessor executes instructions in the form of applications software stored in the memory to perform required processes, such as to cause rotation of the rotary cartridge. Thus, actions performed by a processing system are performed by the processor in accordance with instructions stored as applications software in the memory and/or input commands received via the I/O device, or commands received from other processing systems, such as from a processing system in the AED. The applications software may include one or more software modules, and may be executed in a suitable execution environment, such as an operating system environment, or the like,

Accordingly, it will be appreciated that the processing system may be formed from any suitable processing system, such as a suitably programmed controller, microprocessor, microchip processor, logic gate configuration, firmware optionally associated with implementing logic such as an FPGA (Field Programmable Gate Array), or the like. However, it will also be understood that the processing system could be or could include any electronic processing device such as a tablet, smart phone, computer, or any other electronic device, system or arrangement.

In particular, processing system may automate the apparatus 100 such that the operator is prompted to perform at least some of the steps of the abovementioned method of performing CPR using the apparatus 100. For example, the processing system may prompt the operator to perform at least one chest compression on the subject, for example, using the display and/or an alarm/alert. In this regard, the display may be any suitable display including a screen and/or indicator light, or the like. Additionally and/or alternatively, the processing system may prompt the operator to perform the supply of at least one medicated and/or non-medicated breath to the subject's airway.

Optionally, the processing system may interface to one or more sensors, for example, for sensing signals indicative of vital signs of the subject, such as heart beat, respiration rate, and the like. Additionally or alternatively, the sensors may sense signals indicative of one or more functions of the apparatus 100, such as the administration of a medicated and/or non-medicated breath, the supply of a positive pressure or negative pressure gas to the apparatus 100, or the like. Thus, the signals may be used by the processing system to ensure the apparatus 100 is being used in accordance with the desired method and/or to prompt the operator to perform the next step of the desired method,

For example, in the event the processing system determines that the subject is experiencing cardiac fibrillation, the operator may be prompted by the processing system via the display/alarm to begin performing CPR, and/or the timing and duration of chest compressions, medicated and non-medicated breaths. Alternatively, in the event the processing system determines that a normal heart rhythm has been restored, the operator may be prompted by the processing system via the display/alarm to stop performing CPR on the subject.

As discussed above, the processing system may selectively administer one or more appropriate doses of particulate medicament according to one or more subject characteristics. For example, an infant or child may require a smaller dose and/or fewer doses than an adult In this regard, the subject characteristics may be sensed via one or more sensors, or alternatively may be input by the operator, and the processing system may select the appropriate medicament container 110 containing the desired dose, or alternatively cause metering of an appropriate dose, for administration to the subject based upon the subject characteristics.

Furthermore, the processing system may be in communication with an AED, or any other suitable medical equipment. In this regard, the processing system may interface with a processing system associated with the AED in order to co-ordinate the administration of defibrinating shocks on the subject using the AED and the performance of CPR on the subject using the apparatus 100. Alternatively, a single processing system may be used to control both the AED and the apparatus 100.

In a further example, the processing system may control the rotation of the rotary cartridge in accordance with the method of performing CPR on the biological subject. In this regard, the processing system may rotate the cartridge in accordance with the number of non-medicated breaths to be performed between each medicated breath. Thus, the processing system may determine, for example using signals sensed via the sensors, when artificial breaths of positive pressure gas are supplied to the apparatus 100, and thus rotate the rotary cartridge after zero or more non-medicated breaths, thereby automating the ratio of medicated to non-medicated breaths. This arrangement is particularly beneficial as the operator does not need to manually count or record each time a medicated and/or non medicated breath is delivered to the subject, thus negating the risk of incorrect counting which may lead to harmful over- or under-dosing.

The apparatus 100 may also include a backflow mechanism, such as a one-way valve, or the like, to prevent the particulate medicament from being transferred through the inlet 120. The backflow mechanism thus prevents the flow of medicament out of the inlet 120, which could then be transferred to the operator, for example, in the event the operator's exhalation is providing the positive pressure gas to the apparatus 100. In addition, the backflow mechanism may also ensure that substantially the entire dose is administered to the subject's airway, and is not dissipated or lost, for example, to the external environment.

In a further example, the apparatus 100 may be disposable after the one or more doses of particulate medicament are consumed, expire, or the like. This is beneficial as it allows the operator to merely discard and replace the apparatus 100 as required, rather than needing to reload, disassemble/reassemble the apparatus 100 in order to replace any spent doses of medicament. However, this is not essential, and in other examples the medicament container 110 may be detachable, disposable and replaceable, or alternatively the cartridge containing more than one medicament containers 110 may be detachable, disposable and replaceable.

In some instances, it is preferable to supply the positive pressure gas G in accordance with artificial respiration of the subject. In this regard, the gas G may be supplied (according to the invention) in amounts corresponding to artificial 'breaths', thereby administering the particulate medicament and artificially respirating the subject at the same time, and this will be discussed further below.

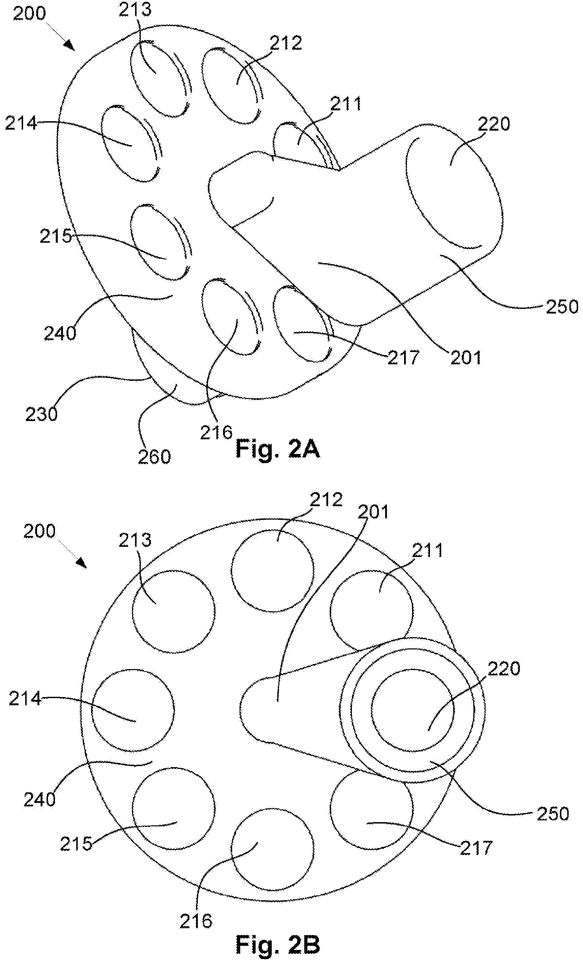

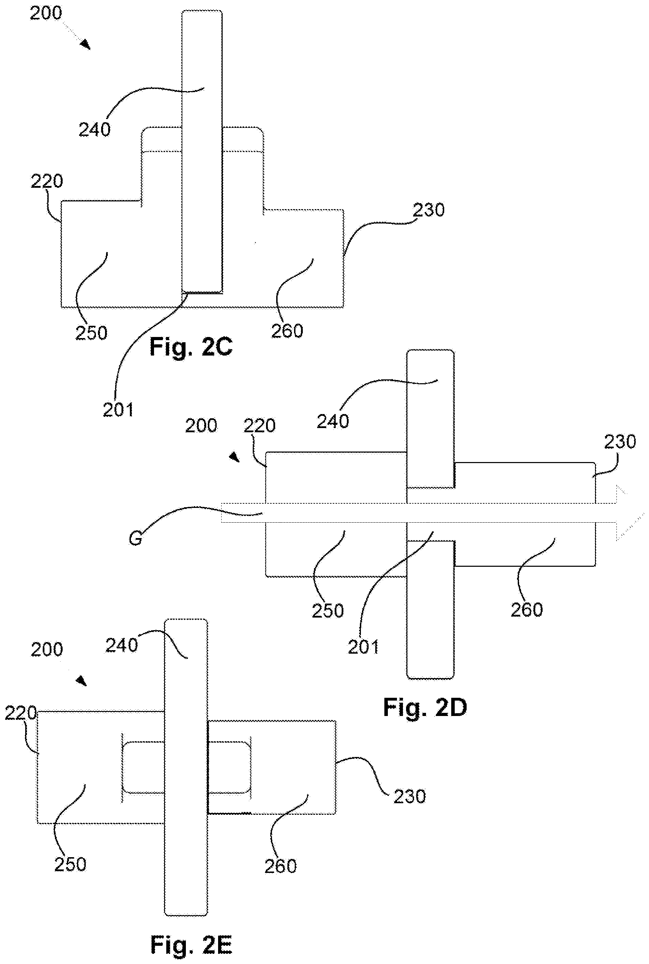

A second example of an apparatus for use in administering a particulate medicament to a biological subject's airway is shown in Figures 2A to 2E. Features similar to those of the example described above have been assigned correspondingly similar reference numerals.

In this particular example, the apparatus 200 is for administering microparticles of adrenalin. Typically the microparticles have a diameter between 1 and 20 µm, more typically between 1.5 and 10 µm, and most typically between 2 and 5 µm. In this respect, the microparticles may be manufactured using any known microparticle technique. Alternatively, the adrenalin may be manufactured as microspheres and/or microcapsules, also using any known microsphere and/or microcapsule technique.

The apparatus 200 includes an inlet 220 and an outlet 230 mounted to a housing 201. The apparatus further includes a rotary cartridge 240 which is rotatably mounted to the housing and includes eight medicament containers 210, 211, 212, 213, 214, 215, 216, 217 that are selectively positionable in a delivery position in which the medicament container 210 is in fluid communication with the inlet 220 and outlet 230. However, as discussed above this is not essential and the apparatus 200 may include any number of medicament containers. In one embodiment, the number of medicament containers 210, 211, 212, 213, 214, 215, 216, 217 corresponds to the recommended number of doses of particulate adrenalin for a subject in cardiac arrest. Each medicament container 210, 211, 212, 213, 214, 215, 216, 217 includes a dose of adrenalin microparticles. Figures 2A and 2B show medicament container 210 in a delivery position, placed between the spaced apart inlet 220 and the outlet 230.

The inlet 220 and the outlet 230 are in fluid communication with the medicament container 210 in the delivery position, so that in use, a positive pressure gas G, is supplied to the subject's airways via the medicament container 210 in order to entrain the adrenalin microparticles in the gas, and thus deliver the entrained medicament to the subject's airway.

In this regard, each medicament container 210, 211, 212, 213, 214, 215, 216, 217 includes two rupturable membranes which, in the delivery position, largely correspond to the shape of the inlet 220 and the outlet 230, as shown in Figure 2B. However, this is not essential and the rupturable membranes may be any suitable shape

As positive pressure gas *G* is supplied to the inlet 220, the rupturable membranes of the medicament container 210 in the delivery position are ruptured under the influence of the gas. Subsequently the adrenalin microparticles are entrained in the gas flow *G* from the inlet 220 to the outlet 230 and thus the gas *G* and adrenalin microparticles are delivered to the subject's airway.

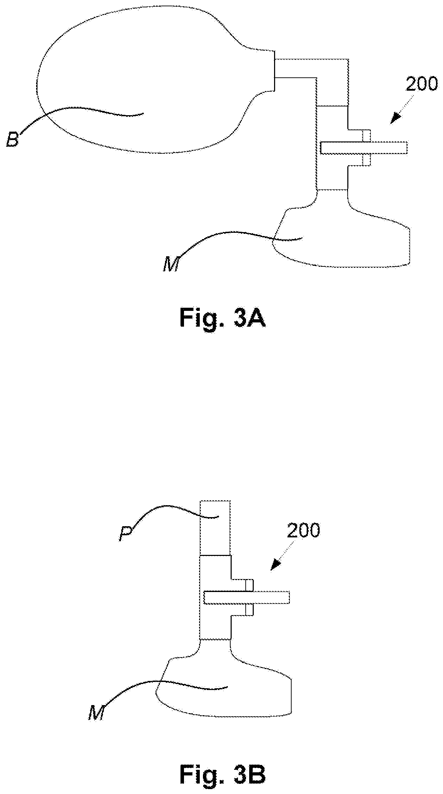

The apparatus 200 further includes an inlet adapter 250 and an outlet adapter 260. In use, the operator may use the inlet adaptor 250 for coupling the apparatus 200 to any suitable positive pressure gas supply, as described above. Additionally or alternatively, the operator may use the outlet adaptor 260 for coupling the apparatus 200 to any suitable outlet device such as a mask and/or endotracheal tube. In this regard, Figure 3A shows an example of the apparatus 200 coupled to a BVM *B* via the inlet adapter 250 for supplying gas to the apparatus 200, and coupled to a mask *M* via the outlet adaptor 260 where the mask is for placing over the subject's mouth. Figure 3B shows an example of the apparatus 200 coupled to a mouthpiece *P* via the inlet adaptor 250, where an operator may use the mouthpiece *P* for supplying positive pressure gas to the inlet of the apparatus 200. The apparatus 200 in Figure 3A is also coupled to a mask *M* via the outlet adaptor, as discussed above.

The operator may alternatively use the inlet adapter 250 as a mouthpiece for exhaling into in order to supply gas. Additionally or alternatively, the outlet adapter 260 may be inserted into or provided over the subject's mouth such that the entrained adrenalin particles are supplied to the subject's airway via the outlet adapter 260.

Gas under negative pressure rotates the rotary cartridge, thus providing a next medicament container in the delivery position between the inlet 220 and the outlet 230. Thus, inflation of the BVM or the like causes the rotary cartridge to advance, and provides the next medicament container ready to be administered to the subject. This ensures that the apparatus 200 is particularly easy to use and that the doses of adrenalin may be delivered in quick succession.

Thus, it will be appreciated that the apparatus 200 may be used when performing CPR, or any other type of artificial respiration, on a subject. In this regard, the gas is supplied in amounts corresponding to artificial breaths such that the particulate adrenalin is delivered to the subject when the operator administers the breath to the subject, for example, via the operator exhaling into the inlet 220, or using a BVM in fluid communication with the inlet 220, or similar. Furthermore, the operator does not require any specialised training in the administration of the adrenalin, as the next medicament container is advanced to the delivery position automatically, for example, when the operator inhales, or the BVM reinflates. Hence the apparatus 200 allows for on-site administration of adrenalin to a subject undergoing cardiac arrest, which in turn improves life expectancy and post-resuscitation outcomes.

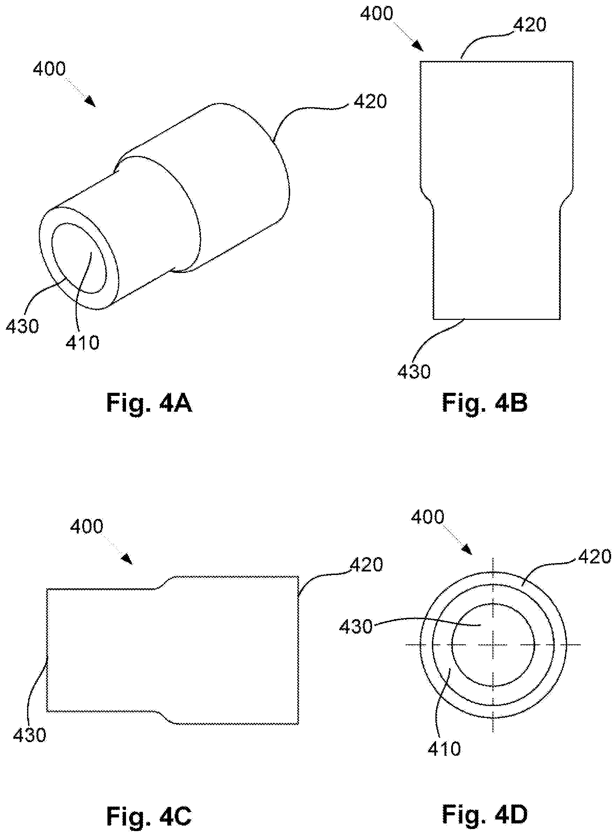

A further example of an apparatus for use in administering a particulate medicament to a biological subject's airway is shown in Figures 4A to 4D. Features similar to those of the example described above have been assigned correspondingly similar reference numerals.

In this example, the apparatus 400 includes a medicament container 410 in fluid communication with the inlet 420 and outlet 430, In particular, the medicament container 410 includes a channel, where particulate medicament is provided at least partially on an inner surface of the channel. Typically the medicament container 410 includes a single dose of particulate medicament, and this arrangement has the benefit of reducing the risk of overdosing the subject. However in some examples the container 410 may contain more than one dose, for example, by providing a thicker coating of medicament on the inner surface of the channel.

It will be appreciated that the simplicity of this apparatus 400 is particularly advantageous for its provision in first aid kits, and for travelling, in the home, office, or the like. In this regard, in use an operator simply positions the outlet 430 in fluid communication with a subject's airway, and exhales into the inlet 420 thereby administering the medicament. However, this is not essential and the apparatus 400 may be used in any suitable environment, including with a BVM, mask, mouthpiece, or the like.

In one example, the apparatus 400 includes a containment mechanism which prevents the medicament from dispersing into the surrounding environment when the apparatus 400 is not in use. It will be appreciated that the containment mechanism may include any arrangement for substantially sealing the medicament container 410 when not in use, such as one or more peelable lids, rupturable membranes, removable caps, pressure operated valves, or the like.

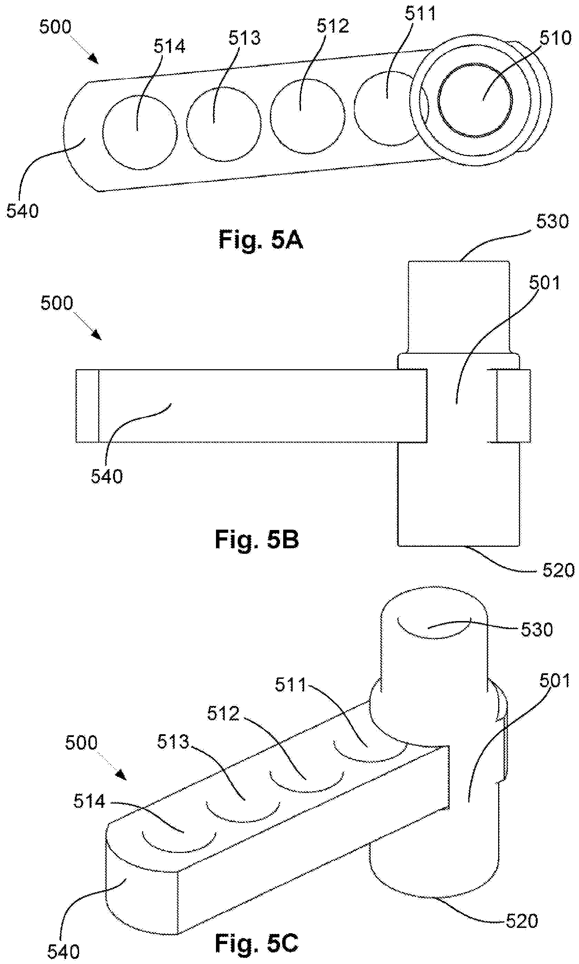

A further example of an apparatus for use in administering a particulate medicament to a biological subject's airway is shown in Figures 5A to 5C. Features similar to those of the example described above have been assigned correspondingly similar reference numerals.

The apparatus 500 in this example includes an inlet 520 and an outlet 530 mounted to a housing 501. The apparatus further includes an elongate cartridge 540 which is movably mounted to the housing and includes five medicament containers 510, 511, 512, 513, 514 that are selectively positionable in a delivery position, in which the medicament container 510 is in fluid communication with the inlet 520 and outlet 530. Each medicament container 510, 511, 512, 513, 514 includes a dose of particulate medicament. Figures 5A, 5B and 5C show medicament container 510 in a delivery position, placed between the spaced apart inlet 520 and the outlet 530.

The inlet 520 and the outlet 530 are in fluid communication with the medicament container 510 in the delivery position, so that in use, a positive pressure gas *G,* is supplied to the subject's airways via the medicament container 510 in order to entrain the particulate medicament in the gas, and thus deliver the entrained medicament to the subject's airway.

As described in other examples above, each medicament container 510, 511, 512, 513, 514 includes two rupturable membranes which, in the delivery position, largely correspond to the shape of the inlet 520 and the outlet 530. As positive pressure gas *G* is supplied to the inlet 520, the rupturable membranes of the medicament container 510 in the delivery position are ruptured under the influence of the gas. Subsequently the medicament is entrained in the gas flow *G* and delivered to the subject's airway.

The elongate cartridge 540 may moved such that hence the next medicament container is positioned in the delivery position, and this may be achieved in any suitable manner including automatically under the influence of negative pressure. Alternatively the operator may manually move the elongate cartridge 540 with respect to the housing 501, for example, by sliding the cartridge 540. Movement of the cartridge 540 may also be controlled by a processing system, such as described above.

The medicament containers 510, 511, 512, 513, 514 may also be removable and/or replaceable, such that the operator may quickly and easily administer further doses of medicament by replacing individual medicament containers 510, 511, 512, 513, 514. Alternatively, the elongate cartridge 540 may be removable/replaceable to rapidly administer any number of doses to the subject, for example, by sliding the cartridge 540 from the housing, and sliding a new cartridge into the housing. However, this is not essential and in other examples the apparatus 500 may be disposable.

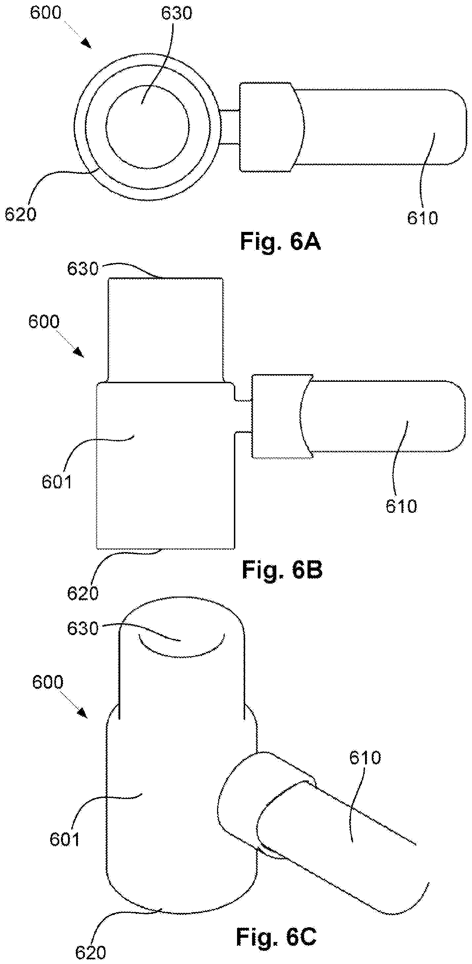

A further example of an apparatus for use in administering a particulate medicament to a biological subject's airway is shown in Figures 6A to 6C. Features similar to those of the example described above have been assigned correspondingly similar reference numerals.

In this example, the apparatus 600 includes a medicament container 610 in fluid communication with a housing 601 including an inlet 620 and an outlet 630. The medicament container 610 may include a single dose of particulate medicament, however typically includes a number of doses.

In use, in one example as positive pressure gas flows from the inlet 620 to the outlet 630, the operator may actuate the medicament container 610 to release a dose of medicament into the gas flow to thereby entrain the medicament and administer it to the subject's airways. In this respect, the container 610 may be actuated in any suitable manner to release a dose of medicament, for example by depressing the container 610, similar to the canister on a metered dose inhaler, or by actuating a button, or the like. Alternatively, as positive pressure gas flows from the inlet 620 to the outlet 630, a dose of medicament may be entrained into the gas flow via the Venturi effect, as described above.

In this regard, the apparatus 600 provides a small and simple device for rapidly administering medicament to a subject's airways, where the medicament container 610 can be quickly removed and replaced when required. Furthermore, the apparatus 600 is sufficiently portable to be provided in small first aid kits, or as a standalone device in handbags, schoolbags, or the like. In addition, as discussed above, the apparatus 600 may be used with a BVM, mouthpiece, mask, or the like, thus providing a portable and versatile arrangement.

It will be appreciated that the apparatus 600 may be used with a number of different particulate medicaments, for example, such that a medicament container 610 containing adrenalin may be used with the apparatus 600 for subjects with cardiac dysfunction or anaphylaxis, or a container 610 containing glucose may be used with the same apparatus 600 in the event a subject is hypoglycaemic. Thus, the apparatus 600 may be provided in a kit with a number of different medicament containers 610 containing different medicaments, such that the operator or subject may select the appropriate container 610 depending upon the subject's condition.

In one example, the medicament container 610 includes particulate medicament which is at least partially compressed. In this regard, the medicament container 610 may be provided separately to the apparatus 600, and in use the operator positions and/or depresses the container 610 in the apparatus 600, which causes the medicament container 610 to open such that the particulate medicament at least partially disperses into the housing 601. In this regard, the medicament container 610 may be opened in any suitable manner and in one example the container 610 includes a plug which is displaced by the apparatus 600 upon attachment thereto and/or depression of the container 610 against the housing 601.

Thus, as positive pressure gas is supplied to the apparatus 600 the medicament which has dispersed into the housing 601 is entrained in the gas flow and delivered to the subject's airways. In this regard, the degree of compression of the medicament in the medicament container 610 may at least partially influence the dose delivered to the subject. In this respect, the medicament container 610 may include a single dose which is highly compressed and thus rapidly dispersed into the housing 601, or multiple doses which are partially compressed and therefore slowly released into the housing 601 for delivery to the subject over multiple artificial breaths.

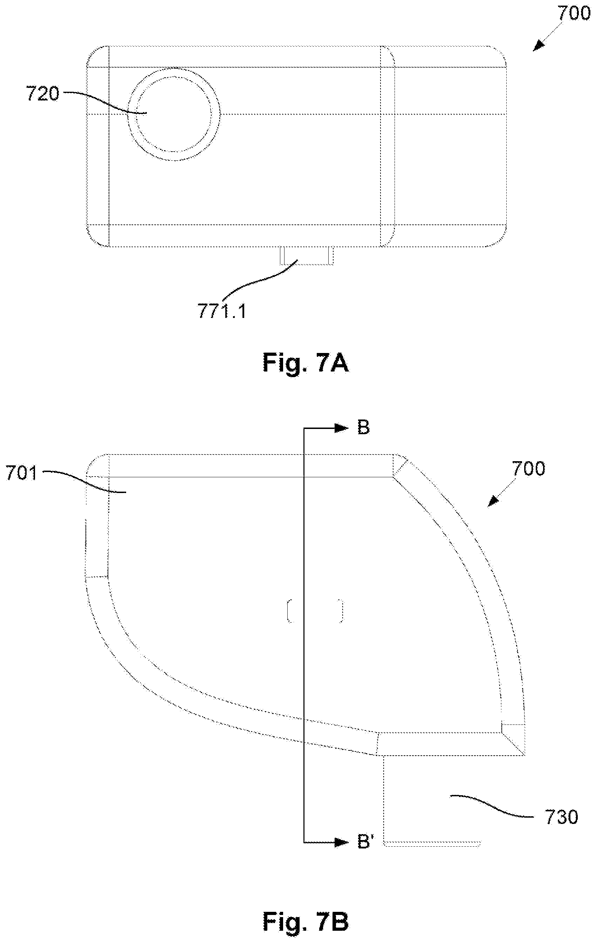

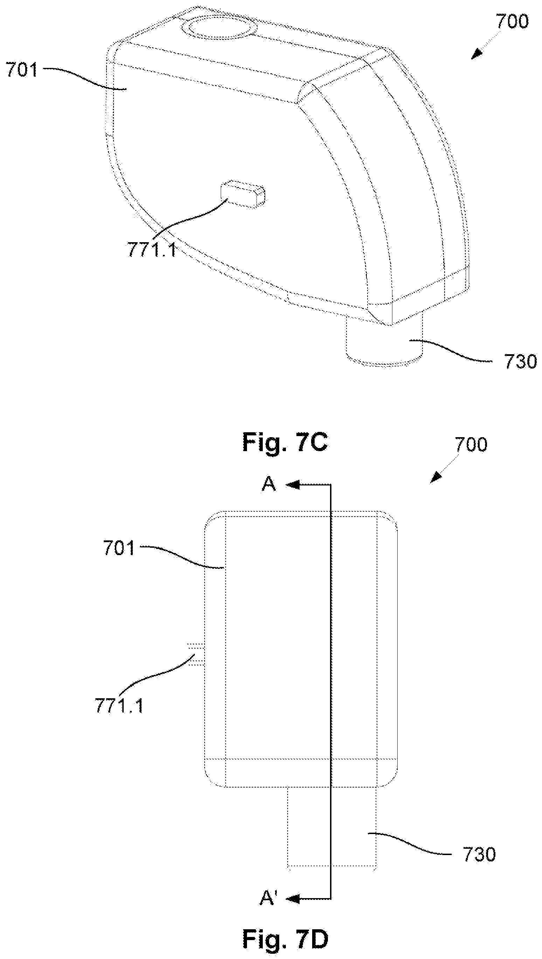

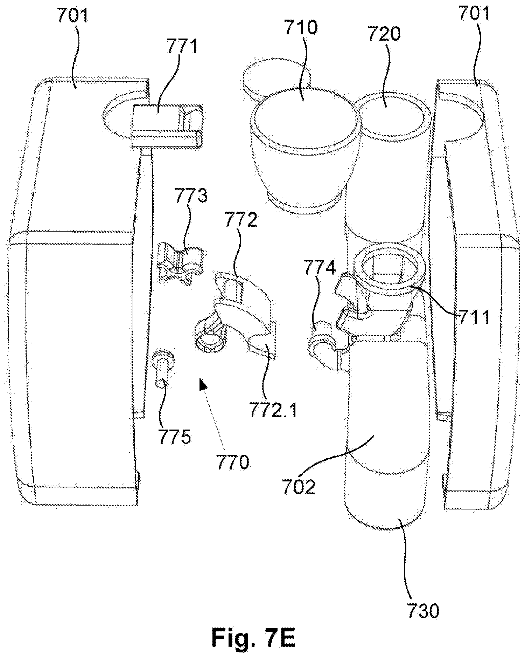

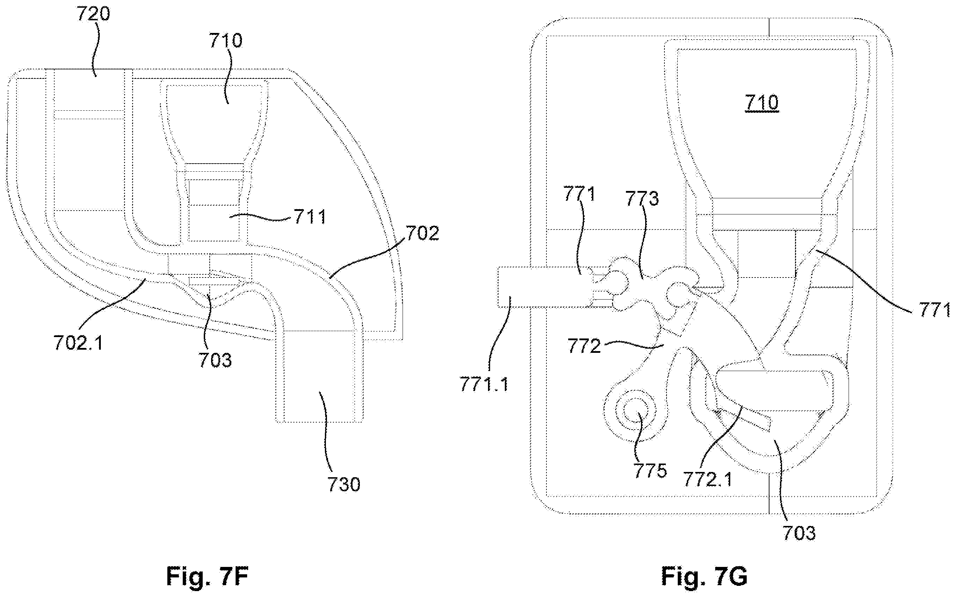

A further example of an apparatus for use in administering a particulate medicament to a biological subject's airway is shown in Figures 7A to 7I. Features similar to those of the example described above have been assigned correspondingly similar reference numerals,

In this example, the apparatus 700 includes a housing 701 defining an inlet 720 and an outlet 730 interconnected via an "S"-shaped channel 702 so that the inlet 720 and outlet 730 are laterally offset. The apparatus also includes a medicament container in the form of a reservoir 710 positioned above and in fluid communication with the channel 702 via a reservoir outlet 711. An actuator 770 is provided to selectively open the reservoir outlet 711 to thereby control dispensing of medicament into the channel 702.

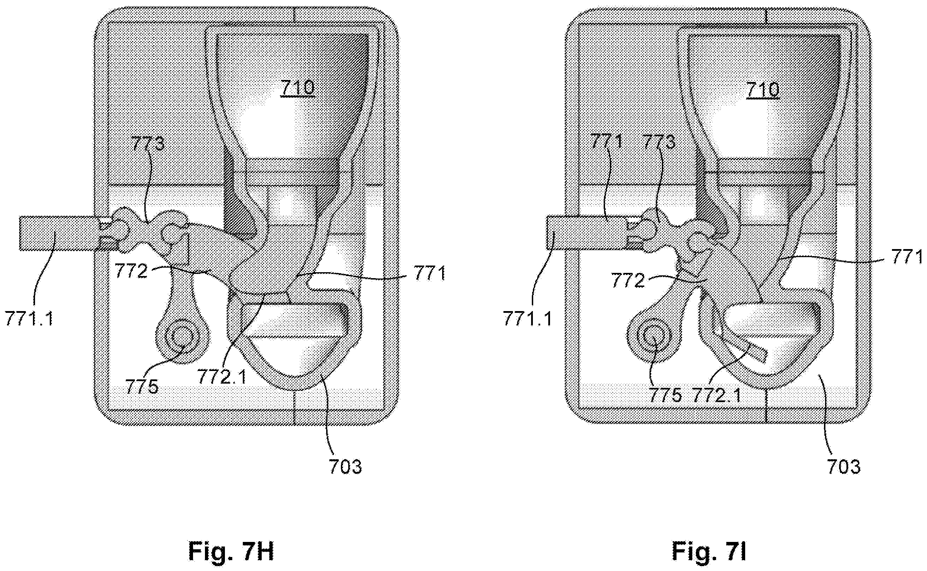

In this regard, the actuator 770 is provided including an arm 771 coupled to a dosing member 772, via a connector 773. The dosing member 772 includes a cup 772.1 and is pivotally mounted to a support 774 via a fastener, such as a bolt 775, allowing the dosing member 772 to be pivoted between loading and dispensing positions shown in Figures 7H and 7I, respectively.

In use, the dosing member is biased by a spring or other biasing member (not shown) into the loading position, with the cap 772. 1 closing the reservoir outlet 711 so that the cup fills with particulate medicament from the reservoir 710, An end of the arm 771 projects outwardly from the housing 701 to form a button 771.1, so that when the user depresses the button, the dosing member 772 pivots into the dispensing position. As this occurs, the cup 772.1 rotates, with the reservoir outlet 711 scraping excess particulate medicament from the cup 772.1, thereby dispensing a defined volume of medicament into the channel 701.

The dosing member 772 is positioned in an upper surface of a laterally extending channel portion 702.1 of the channel 702, and is further positioned above a recess 703 so that medicament is supplied into the recess 703 of the channel portion 702.1 in use. This ensures that medicament rests within the recess 703 and does not fall directly out of the outlet 730 and into the subject's reservoir outlet in use.

Accordingly, in use, the outlet 730 would typically be positioned in the subject's mount and the button 771.1 pushed to dispense particulate medicament into recess 703 within the channel 702. Positive gas pressure is then applied to the inlet 720, thereby generating gas flow through the channel 701, with turbulence in the vicinity of the recess 703 causing the particulate medicament to become entrained within the gas flow, thereby delivering the medicament to the subject's lungs.

The apparatus can interface directly with Ambu-bag and fish-reservoir outlet value through the device inlet, and allows for metered doses that are 'sealed' until delivery. The delivery set up requires one action of pressing a button before pumping the Ambu-bag. The system immediately sets itself up for the next dose administration. The device is compact and can contain dozens of ddoses, with multiple dosing options being available through the use of different sized arms. Additionally, the arrangement provides an unobstructed airway regardless of whether or not medicament is being delivered.

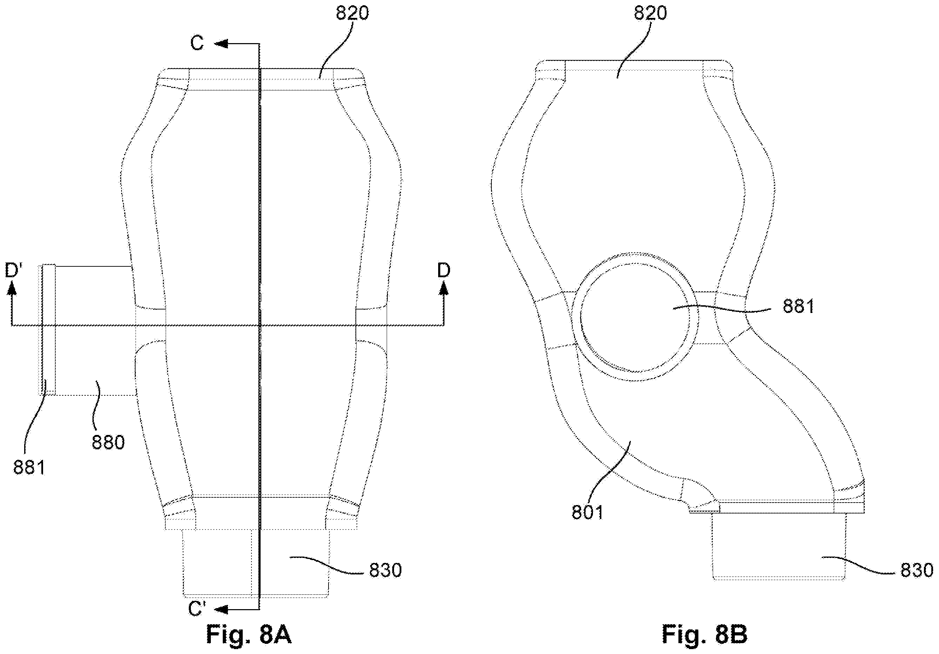

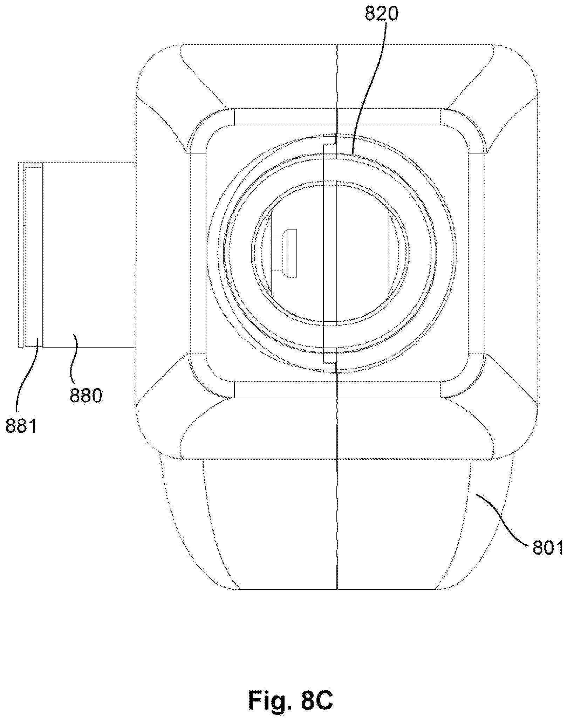

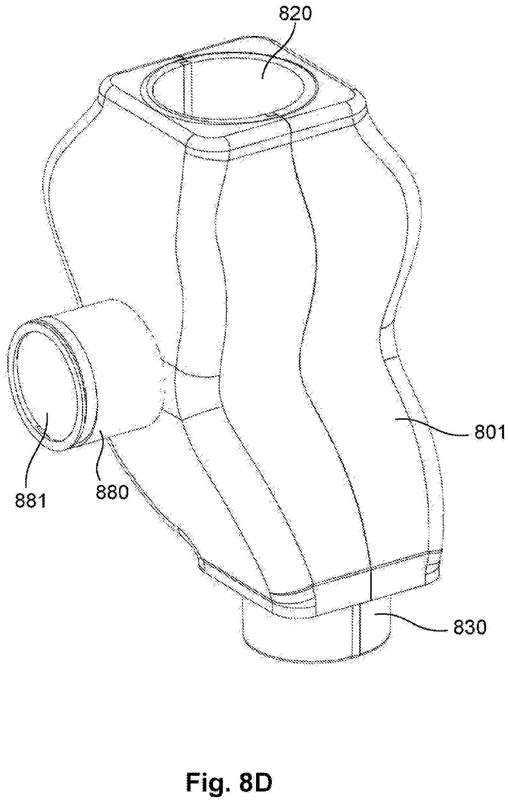

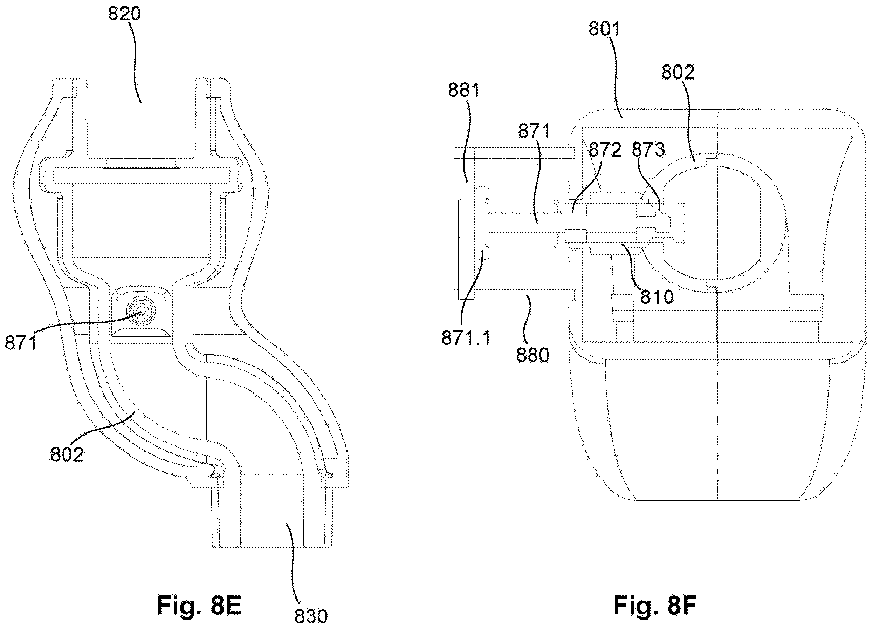

A further example of an apparatus for use in administering a particulate medicament to a biological subject's airway is shown in Figures 8A to 8F. Features similar to those of the example described above have been assigned correspondingly similar reference numerals.

In this example, the apparatus 800 includes a housing 801 defining an inlet 820 and an outlet 830 interconnected via an "S"-shaped channel 802 formed by a tube within the housing 801, so that the inlet 820 and outlet 830 are laterally offset. The apparatus also includes a medicament container in the form of a tube 810 extending laterally from the channel 802. The tube includes an actuator 870 including an arm 871 extending from the end of the tube 810 to form a button 871.1. A piston ring 872 and stopper 873 are mounted spaced apart on the arm 871, to define an annular cavity therebetween for containing the particulate medicament. In use, depressing the button 871.1 urges particulate medicament out of the tube 810 and into the channel 802, whilst the stopper 873 engages the inside of the channel 802 preventing further movement of the arm 871.

In this example, the apparatus further includes a cover tube 880 and lid 881, which protect the button 871.1 and prevent accidental actuation. In use, the lid 881 can be removed allowing the button to be pushed as required.

In this example, pressing the button releases a single dose of particulate medicament retained within the tube 810. It will be appreciated however that a dosing arrangement could also be used to place a single dose of particulate medicament within the tube 810 from a reservoir, thereby allowing a multi-dose configuration to be provided. Alternatively, multiple cavities could be defined along the length of the arm using multiple spaced apart rings 872. In this case, the button would be pushed until a catch engages, to release the first dose of medicament, with further pushes being used to release further doses as required.

It will also be appreciated that in the above example, the medicament container 610 could be selectively closable to prevent additional medication being dispensed when not required, or alternatively could be interchanged for an empty container.

In one example, the apparatus 100, 200, 400, 500, 600, 700, 800 of any of the above examples may be provided in a kit, which additionally includes any one or more of a mask, a BVM, an Ambu bag, a defibrillator, different models of the apparatus, additional apparatus and/or replacement medicament containers and/or replacement rotary cartridges, and the like.

It will be appreciated that the apparatus 100, 200, 400, 500, 600, 700, 800 of any one of the above examples may also be used (not according to the invention) with negative pressure gas, such that as gas under negative pressure flows from the inlet to the outlet, the particulate medicament is entrained in the gas flow and delivered to the subject's airway. In particular, the negative pressure gas may be supplied by the subject, and this is particularly suited to situations where the subject is conscious and responsive. For example, a subject suffering anaphylaxis or hypoglycemia may utilise the apparatus 100, 200, 400, 500, 600, 700, 800 quickly and effectively in order to rapidly administer themselves with one or more doses of adrenalin or glucose, respectively, in order to treat their condition and avoid further deterioration.

Furthermore, the apparatus 100, 200, 400, 500, 600, 700, 800 is highly portable, such that a subject prone to any one of the above conditions would be able to carry the apparatus with them, for example, in a pocket, handbag, purse, schoolbag, or the like. Additionally, as the apparatus 100, 200, 400, 500, 600, 700, 800 may be used with positive or negative pressure, in the event a subject deteriorates to a state of unresponsiveness, an operator may use the apparatus 100, 200, 400, 500, 600, 700, 800 to administer further doses of the medicament.

Thus, it will be appreciated that the abovementioned examples include a method (not according to the invention) and apparatus for use in administering a particulate medicament to a biological subject's airway, and a method for performing CPR on a biological subject, which include advantages such as portability, lower cost, negate the need for specialised training and save time thus greatly improving the subject's prognosis.

### Experimental

The effectiveness of the above described techniques for delivering adrenalin were tested using a sheep model. The procedure of the experiment will be as follows.
1. The animal is immobilized and brought into the preparation room in a sling cage to allow intravenous access to be gained.
2. The animals face, neck and left chest wall and abdomen are shaved,
3. A multi-lumen central venous catheter and an 8G sheath are inserted into the left jugular vein of the animal under local anesthetic and sutured into place,
4. Increments of midazolam (5mg) are administered via central line or intramuscular injection to maintain sheep comfort during the prolonged procedure, whilst avoiding excessive sedation. Animal must be able to maintain own airway.
5. An anesthetic induction dose of buprenophine 0.01.ing/kg, midazolam 0.5mg/kg and alfaxalone 3mg/kg is given. It is from this point on that the animal remains unconscious and under general anesthetic until the end of the study period.
6. The animal is intubated with a size appropriate orotracheal tube (#8.0, 9.0 or 10.0) and then brought into theatre and transferred to the operating table. Positioning sheep in the left lateral position.
7. Mechanical ventilation is commenced. Respiratory rate - 12 breaths/min, tidal volumes 10mls/kg, PEEP 10cmH2O, Pkp <35 and FiO2 0.21 - ventilator setting will be titrated to maintain PCO2 35-45 and PaO2 >70. Capnography is monitored with ventilation.
8. Maintenance anesthesia is then be commenced with an infusion of alfaxalone 6mg/kg/hr, midazolam 0.7mg/kg/hr, fentanyl 50mcg/kg/hr and ketamine 8mg/kg/hr. This is adjusted according to biological parameters (BP, HR, CVP and sedation) to ensure a constant surgical plane throughout the study period until euthanasia.
9. An infusion of Hartmann's solution will be commenced via the central venous line at 10mL/kg/hr for maintenance.
10. Commence monitoring.
   i. A pulse oximeter is used to monitor SpO₂.
   ii. An ECG is attached
11. Pulmonary artery catheter, which can facilitate cardiac pacing is inserted into the left IJ sheath.
12. A urinary catheter is inserted and attached to a urinary catheter bag.
13. Orogastric tube (Salem Sump 14Fr) is inserted and sutured to lip and put on free drainage.
14. The facial artery is identified, dissected out and cannulated with a 20G Vygon catheter. Arterial blood pressure monitoring is commenced via this line.
15. Baseline parameters are measured and recorded.
   i. Continuous monitoring of physiological parameters is attended throughout the protocol. This includes the ventilator settings, ETCO₂, HR, BP, CVP, PAP, cardiac output, SpO₂, temperature, BSL and 15 minutely arterial blood gases.
16. Blood samples are taken via the facial arterial line.
17. Lung protective ventilation will continue (ARDS-Net). Keep ventilator settings standard and titrate only to achieve gas exchange parameters PCO2 35-45 and PaO2 >70.
18. Perform a left thoracotomy, at the fifth intercostals space, exposing the left ventricular free wail.
19. Insert epicardial pacing wires.
20. Cardiac arrest is induced by purpose built fibrillator.
21. Ventricular tachycardia then ventricular fibrillation will follow, finally the heart will descend into asystole.
22. At the point where the animal becomes asystolic, advanced life support commences as per Australian Resuscitation Council Advance Life Support Algorithm.
23. Animal is removed from the ventilator and oxygenation and manual inflation with an ambu bag will be commenced - as per standard clinical procedure for an arresting clinical (human) patient.
*24. TREATMENT ANIMAL ENDOBRONCHIAL-* The adrenaline is administered via the endotracheal tube (adrenaline in liquid form squirted into ETT) - after a 5 minute delay. This is repeated every two (2) minutes as per ALS algorithm for three (3) cycles - doses 2.5mg/cycle (one dose per cycle).
25. *TREATMENT ANIMAL FOR CURRENT SYSTEM-* The adrenaline respule is attached and administered via the endotracheal tube - this is repeated every two (2) minutes as per ALS algorithm for three (3) cycles - doses 2.5mg low dose study and 10mg high dose sty (one dose per cycle).
26. *TREATMENT ANIMAL INTRAVENOS* - Adrenaline is administered intravenously via the central venous line - after a 5 minute delay. This is repeated every two (2) minutes as per ALS algorithm for three (3) cycles - dose 1mg each cycle in line with current practice.
27. *CONTROL ANIMAL* - CPR and manual ventilation continue through study period without any adrenaline administration. 5 minutes of CPR will elapse followed by x3 2 minutes cycles.
28. *SHAM ANIMAL* - Instrumentation only. No cardiac arrest administered. Animal ventilated and sedated throughout study period.
29. Serial blood samples are attended at 1 minutely intervals to measure adrenaline levels:
30. General anesthetic continues as a continuous infusion as per rates mentioned previously, titrated to effect. Animals kept comfortable at all times.
31. At completion of experiment (after three cycles of advanced life support) animals is humanely killed with sodium pentobarbitone 295mg/mL, 0.5 mL/kg.
32. The loss of electrical activity in the heart, loss of blood pressure and loss of cardiac output will confirm death.

The results of blood plasma analysis are shown in Tables 1 to 6 below. Table 1 shows use of the device with n=3 and an adrenaline dose 10mg, Table 2 shows use of the device with n=3 and an adrenaline dose of 2.5 mg, Table 3 shows use of intravenous delivery with n=3 and an adrenaline dose of 1mg, Table 4 shows use of endobroncial delivery with n=3 and an adrenaline dose of 2.5mg, Table 5 shows controls with n=2 and no adrenaline given and Table 6 shows sham animals n=2 with no adrenaline given and no cardiac arrest induced.

**Table 1**

| **Device** | **1** | **2** | **3** |
|---|---|---|---|
| **B** | <0.1 | 0.8 | 0.8 |
| **T1** | 0.2 | 0.6 | 0.1 |
| **T2** | <0.1 | 0.9 | 0.5 |
| **T3** | 0.2 | 1 | 0.2 |
| **1M** | 105 | 208 | 1090 |
| **2M** | 611 | 736 | 1780 |
| **3M** | 741 | 1620 | 2370 |
| **4M** | 734 | 3300 | 2850 |
| **5M** | 1840 | 3160 | 3560 |
| **6M** | 2360 | 4040 | 4810 |

**Table 2**

| **Device** | **1** | **2** | **3** |
|---|---|---|---|
| **B** | 0.1 | 1 | Not done |
| **T1** | 0.4 | 0.4 | 0.4 |
| **T2** | 0.2 | 0.4 | 0.9 |
| **T3** | 0.3 | 0.1 | 0.9 |
| **1M** | 1.8 | 5.3 | 8.4 |
| **2M** | 22 | 25 | 37 |
| **3M** | 38 | 38 | 116 |
| **4M** | 103 | 49 | 214 |
| **5M** | 219 | 60 | 411 |
| **6M** | 307 | 71 | 542 |

**Table 3**

| **Intravenous** | **1** | **2** | **3** |
|---|---|---|---|
| **B** | 3.9 | 0.8 | <0.1 |
| **T1** | 3.5 | 0.9 | 1,9 |
| **T2** | 2.6 | <.1 | 1.3 |
| **T3** | 126 | 21 | 48 |
| **1M** | 138 | 60 | 2020 |
| **2M** | 647 | 743 | 9400 |
| **3M** | 2050 | 2960 | 3340 |
| **4M** | 10070 | 6070 | 10300 |
| **5M** | 13240 | 3280 | 7950 |
| **6M** | 15800 | 6310 | 11080 |

**Table 4**

| **Endobroncial** | **1** | **2** | **3** |
|---|---|---|---|
| **B** | 2.4 | 4.6 | 0.3 |
| **T1** | 1.3 | 1.6 | 0.4 |
| **T2** | 1.4 | 2.4 | 0.4 |
| **T3** | 535 | 554 | 176 |
| **1M** | 352 | 1020 | 63 |
| **2M** | 324 | 1070 | 62 |
| **3M** | 230 | 998 | 73 |
| **4M** | 231 | 889 | 123 |
| **5M** | 298 | 934 | 224 |
| **6M** | 362 | 1040 | 212 |

**Table 5**

| **Control** | **1** | **2** |
|---|---|---|
| **B** | 0.8 | 2.8 |
| **T1** | 0.6 | 0.3 |
| **T2** | 0.6 | 0.3 |
| **T3** | 165 | 35 |
| **1M** | 166 | 12 |
| **2M** | 151 | 8,2 |
| **3M** | 120 | 19 |
| **4M** | 107 | 2.6 |
| **5M** | 95 | 16 |
| **6M** | 75 | 30 |

**Table 6**

| **Sham** | **1** | **2** |
|---|---|---|
| **B** | 2.3 | 1.6 |
| **T1** | 0.3 | 0.1 |
| **T2** | 0.2 | 0.3 |
| **T3** | 0.5 | 0.4 |
| **1M** | 0.3 | 0.4 |
| **2M** | 0.2 | 13 |
| **3M** | 0.4 | 0.3 |
| **4M** | 0.6 | 21 |
| **5M** | 0.2 | 0.2 |
| **6M** | 0.2 | 0.3 |

Results show a marked increase in blood plasma adrenalin levels using the device, better than that obtained for endobronchial delivery. Whilst the increase was not as marked as with intravenous injection, this was as expected and in any event, avoids the drawbacks of intravenous injection.

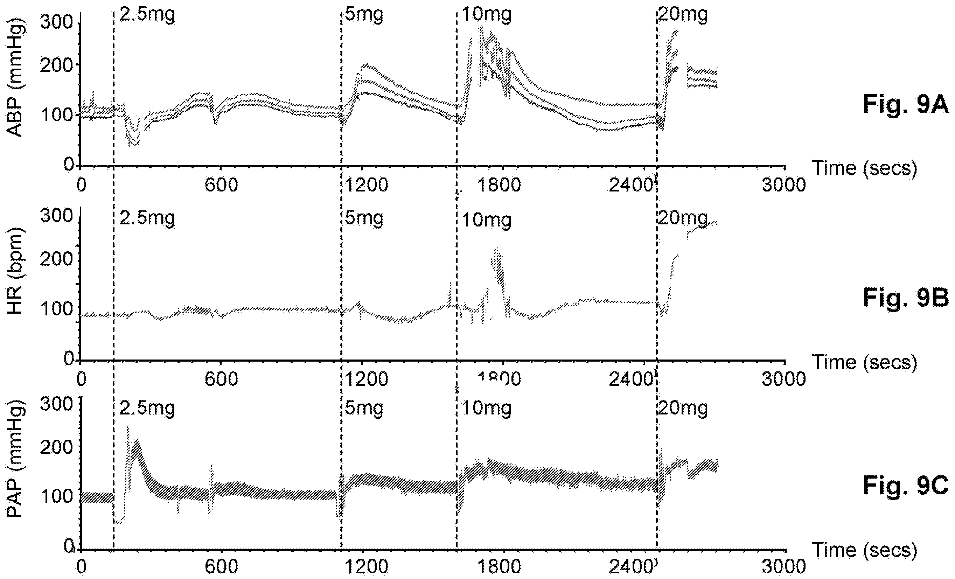

A further test was performed involving measuring blood parameters, including ABP, HR and PAP during sequential delivery of 2.5mg, 5mg, 10mg and 20mg of particulate adrenalin to a sheep. Graphs of the results are shown in Figures 9A to 9C, which highlight the increase in ABP, HR and PAP immediately post-delivery, highlighting the ability of the techniques to produce immediate benefits to a patient.

In order to demonstrate the ability to deliver glucose, it is necessary to be able to create glucose particles of suitable dimensions for delivery through the airway.

An attempt to achieve this via spray drying involved creating a D-glucose solution in water (15 mg/ml) spray drying this in an open loop arrangement (air as the drying gas) at a feed rate of 2 ml/min, aspiration rate of 38 m³/h and atomization rate of 819 NL/h. The inlet and outlet temperatures were 80°C and 56°C, respectively. Re-crystallisation of powder took place in the collection pot instantaneously during the spray drying process. Therefore, a stable glucose powder formulation cannot be prepared by direct spray drying of the glucose solution.

As an alternative, jet milling was proposed with D-glucose powder being milled at the 4-5 bar feed pressure and 3-4 bar grinding pressure with a feed rate setting of 10%. Particle size measured by laser diffraction technique, shown in Table 7 below, indicates that the milled powder has significantly reduced in size and now are in respirable range. In this regard, anything below 5 micrometres in size can be delivered to alveolus, and hence be absorbed into the blood.

**Table 7**

| Formulation | D₁₀ (µm) | D₅₀ (µm) | D₉₀ (µm) | Span (D₉₀-D₁₀)/D₅₀ |
|---|---|---|---|---|
| Before milling | 11.60 | 191.68 | 404.10 | 2.048 |
| After milling | 0.68 ± 0.04 | 1.76 ± 0.05 | 3.79 ± 0.08 | 1.77 ± 0.03 |

Accordingly, the above described arrangement provides a mechanism to deliver particulate medication directly to a subject's lungs, even in the event that the subject is not themselves able to inhale. In this regard, the lung has a vast blood supply and has been used for drug delivery eg salbutamol, and more recently for some antibiotics to maximise dose delivery to lung. There is only a 2 cell barrier from the alveolus and the blood compartment in the massive capillary network that exists in mammalian lungs. The efficacy of this route of drug delivery can be easily witnessed when assessing the rapidity of onset of action of salbutamol in a sick asthmatic (if they can move enough air). Perhaps more obvious is watching the effect of nicotine or heroin when it is smoked or inhaled. It is therefore clear that the lung is an efficacious drug delivery route.

Delivery of micro-particle adrenaline via forced inhalation allows this life-saving drug to be delivered more readily, and does not require specialised training to administer. Due to vast alveolar perfusion, and the close proximity of alveolar vessels to central cardiac circulation, with only 2 cells between alveolar contents and blood cells, adrenaline inhalation dramatically reduces the latency from administration to therapeutic levels.

The potential impact of this research is far reaching. This device can be placed in key locations, (eg. schools and ambulances), allowing immediate adrenalin delivery in the community including rural areas with minimal professional healthcare staff, military, and shopping centres etc.

Current data demonstrates poor outcomes from community arrests when compared to cardiac arrests that occur on the ward. Achieving IV access in the field, particularly in cardiac arrest is extremely difficult due to lack of circulation and environmental factors e.g. light, position of patient etc. As Such, this technology may result in a significantly higher proportion of these patients having received timely treatment prior to presentation to hospital, and with a greater percentage of these patients surviving to discharge. In future, this technology could be used to study numerous other interventions aimed at early administration without the delays or risks associated with establishing intravenous access.

Recent data from Seattle showed a rising trend of cardiac arrest survival in Seattle & King County, with as many as 55% of cardiac arrests surviving to discharge in 2012. This is in stark contrast to local data, which shows that cardiac arrest survival rates decreased from 12.3% in 2004-2005 to 10.2% in 2009-2010 in NSW, and a mean survival rate of 5% in Queensland out-of-hospital arrests. This discrepancy can mostly be attributed to how successfully the cycle of care is executed in the community (early recognition & notification, early CPR, early defibrillation, and post resuscitation care). A non-invasive, easy to use device to allow adrenaline administration could assist in improving local survival rates. It will also free up skilled team members to concentrate on defibrillation and other matters, rather than focus on finding IV access.

In addition, there are circumstances where there is a delay in establishing intravenous access even in the emergency department. In these situations, this technology could provide ambulance and emergency departments an alternative administration method that can be commenced the moment the patient arrives. This is in contrast to the current alternatives of intraosseous administration and endobrochial, which is typically reserved as a "last resort" due to both being more invasive. Thus, both alternative methods of adrenaline administration are only likely to occur after several minutes of attempting IV access the inhalation/ventilation method could be delivered while the first attempt at IV access is being made. It may augment circulation to a degree where it actually facilitates IV access - in itself a useful and clinically beneficial outcome.

Accordingly, the above described technique provides a device with the potential for safe, rapid delivery of a life-saving drug (adrenaline) without the need for putting a drip in (small plastic tube that sits in a vein) or pouring drugs into the lungs or placing a metal needle directly into the bone, which are alternative methods when a drip cannot be inserted. The device allows rapid delivery of very small particles of adrenaline to be delivered directly to the lungs with each breath This rapid delivery of adrenalin to the lungs gets absorbed through the lung tissue to the blood and then the heart and may help during a heart attack to return to normal heart pumping. Early intervention during a heart, attack could save more lives and avoid poor recovery from a heart attack which sometimes includes brain injury. It is exceptionally simple to use and could be applied in schools, sporting and entertainment venues, ambulances and hospitals amongst other areas. The device could be used by a single person such as a parent, teacher or paramedic to revive an arrested patient. The rapid adrenalin delivery device promises to deliver improved, rapid treatment, and this may translate into better results, to patients throughout the community.

Throughout this specification and claims which follow, unless the context requires otherwise, the word "comprise", and variations such as "comprises" or "comprising", will be understood to imply the inclusion of a stated integer or group of integers or steps but not the exclusion of any other integer or group of integers.

## Claims

1. An apparatus configured to administer microparticles of adrenalin to the lungs of a biological subject to treat anaphylaxis or cardiac arrest, the apparatus including:
a) a supply of positive pressure gas;
b) an inlet adapted for fluid communication with a supply of positive pressure gas;
c) an outlet adapted for fluid communication with the subject's airways to deliver the positive pressure gas to the subject's lungs;
d) a channel extending at least part way between the inlet and the outlet and including at least one baffle for generating turbulent gas flow within the channel;
e) a medicament supply including a dose of the microparticles of adrenalin for release into the channel; and
f) an actuator for selectively supplying the dose of the microparticles of adrenalin into the gas flow, the actuator including a piston for urging the dose of the microparticles of adrenalin into the channel, so that, in use, the dose of the microparticles of adrenalin is entrained in a positive pressure gas flow caused by the supply of positive pressure gas from the inlet to the outlet through the channel,
wherein the apparatus is configured to deliver the dose of the microparticles of adrenalin to the subject's lungs (i) without requiring the subject to inhale and (ii) by an amount of the positive pressure gas flow corresponding to an artificial breath for the subject.

## Patentansprüche

1. Vorrichtung, die dazu eingerichtet ist, Adrenalinmikropartikel an die Lunge eines biologischen Individuums zum Behandeln von Anaphylaxie oder Herzstillstand zu verabreichen, wobei die Vorrichtung beinhaltet:
a) eine Überdruckgaszufuhr;
b) einen Einlass, der zur Fluidverbindung mit einer Überdruckgaszufuhr ausgelegt ist;
c) einen Auslass, der zur Fluidverbindung mit den Atemwegen des Individuums ausgelegt ist, um das Überdruckgas an die Lunge des Individuums abzugeben;
d) einen Kanal, der sich zumindest abschnittsweise zwischen dem Einlass und dem Auslass erstreckt und mindestens eine Schikane zum Erzeugen einer turbulenten Gasströmung innerhalb des Kanals beinhaltet;
e) eine Arzneimittelzufuhr, die eine Dosis der Adrenalinmikropartikel zur Freisetzung in den Kanal beinhaltet; und
f) ein Betätigungselement zum selektiven Zuführen der Dosis der Adrenalinmikropartikel in die Gasströmung, wobei das Betätigungselement einen Kolben zum Drücken der Dosis der Adrenalinmikropartikel in den Kanal beinhaltet, sodass die Dosis der Adrenalinmikropartikel bei Verwendung in eine Überdruckgasströmung mitgenommen werden, die durch die Überdruckgaszufuhr von dem Einlass zu dem Auslass durch den Kanal bewirkt wird,
wobei die Vorrichtung dazu eingerichtet ist, die Dosis der Adrenalinmikropartikel an die Lunge des Individuums abzugeben, (i) ohne dass das Individuum einatmen muss, und (ii) durch eine Menge der Überdruckgasströmung, die einem künstlichen Atemzug für das Individuum entspricht.

## Revendications

1. Appareil configuré pour administrer des microparticules d'adrénaline aux poumons d'un sujet biologique pour traiter une anaphylaxie ou un arrêt cardiaque, l'appareil comprenant :
a) une alimentation en gaz à pression positive ;
b) une entrée adaptée pour la communication fluidique avec une alimentation en gaz à pression positive ;
c) une sortie adaptée pour la communication fluidique avec les voies aériennes du sujet pour distribuer le gaz à pression positive aux poumons du sujet ;
d) un canal s'étendant au moins en partie entre l'entrée et la sortie et comprenant au moins un déflecteur pour générer un flux de gaz turbulent à l'intérieur du canal ;
e) une alimentation en médicaments comprenant une dose des microparticules d'adrénaline à libérer dans le canal ; et
f) un actionneur pour sélectivement alimenter la dose des microparticules d'adrénaline dans le flux de gaz, l'actionneur comprenant un piston pour pousser la dose des microparticules d'adrénaline dans le canal, de telle sorte que, durant l'utilisation, la dose des microparticules d'adrénaline est entraînée dans un flux de gaz à pression positive provoqué par l'alimentation en gaz à pression positive de l'entrée à la sortie à travers le canal,
dans lequel l'appareil est configuré pour distribuer la dose des microparticules d'adrénaline aux poumons du sujet (i) sans nécessiter d'inhalation de la part du sujet et (ii) par une quantité du flux de gaz à pression positive correspondant à une respiration artificielle pour le sujet.
